# EUROPEAN PATENT SPECIFICATION

(11) **EP 0 285 989 B1**
(45) Date of publication and mention of the grant of the patent: **27.10.1993**
(21) Application number: 88105093.4
(22) Date of filing: 29.03.1988
(51) Int. Cl.: A61B 5/103, A43D 1/02

(54) **Apparatus for printing a foot shape**
Gerät zum Abdruck einer Fussform
Appareil pour faire l'empreinte d'une forme de pied

(30) Priority: 06.04.1987 JP 84430/87
(43) Date of publication of application: 12.10.1988
(73) Proprietor: Suzuki, Kazutoyo, Arakawa-ku Tokyo (JP)
(72) Inventor: Suzuki, Kazutoyo, Arakawa-ku Tokyo (JP)
(74) Representative: Körner, Ekkehard, Dipl.-Ing.

(56) References cited:
- WO-A-84/02304
- DE-C- 596 762
- US-A- 4 470 782

## Description

The present invention refers to an apparatus for printing a foot shape.

In order to make a shoe that fits one's foot, it is desirable to use a last having quite an identical shape with the foot. Therefore, shoes were conventionally made by measuring major parts of a foot and, based on the measurement, making a last or choosing the most fitting one from many lasts available.

Although a last corresponds to a foot in the major parts thereof, every foot is of such an individual shape that it is impossible to make a precise last by merely measuring such major parts of the foot and, therefore, some measures for making shoes that are comfortable to wear have been demanded.

From US-A-4,470,782, an apparatus for producing custom orthotics is known, including a boundary wall encompassing a substrate pad and an overlying elastic skin to form an expandable interior chamber into which high density molding material is injected. A matrix of axially movable drive pins project through the pad and are urged upwardly into contact with the arch of the individual's foot to maintain the foot in a properly aligned position during fabrication of the orthotic, the drive pins carrying a support bridge driven into conforming contact against the arch of the foot. When using this apparatus, it is possible to bring the drive pins into predetermined positions and affix them therein which may, in some instances, possibly be higher than that attained by simply stepping onto the pins, i.e., the foot arch may be brought into a corrected position by the pins so as to correct foot defects such as fallen arches.

From WO 84/02304, an apparatus for producing a corrected mold for a foot having a rear portion and a forefoot portion is known, consisting of a resilient cushion having a rear section with a first thickness and a front section with a second, smaller thickness for supporting the foot such that the foot is positioned on the resilient cushion with its forefoot portion being positioned lower than its rear portion thereof and being relatively immobile with respect to its rear portion thereof. The position of the rear portion of the foot on the resilient cushion is adjusted so that the resilient cushion adopts a corrected contour of the foot and then a molding device or material is positioned between the foot and the resilient cushion for producing the corrected mold. The apparatus makes use of a foil which is in a preheated condition when the foot is placed thereon for making a footprint. Cooling of the foil after having printed the foot cures the foil.

DE-C-596 762 discloses an apparatus for reproducing the shape of a human body part, particularly a foot, for orthopedic purposes, comprising a plurality of spring loaded pins projecting through a perforated plate. At the lower ends of the pins wedge-shaped blades are affixed that pierce into a paper foil when a foot steps onto said pins. The lengths of the incisions created by the blades within the paper foil is a measure of the shape of the foot sole.

It is the object of the invention to provide an apparatus for printing a foot shape precisely that is simple in construction and low in cost.

This object is attained by the features of Claim 1. Preferred embodiments of the invention are subject matter of the dependent claims.

According to the apparatus embodying the invention, when stepping on the film, which is plasticized by heating and covers the upper end surfaces of the measuring elements facing the opening, the measuring elements accompanied by the film are lowered in accordance with the indentation in the sole of the foot and the film is printed with a foot shape and subsequently cooled. After removal of the film, the lowered measuring elements are lifted up to their original height positions, i.e., to the same elevation with the top plate surface so as to make them ready for another operation.

When the heating means comprises a heating chamber facing said opening, the film plasticized by heating same can be immediately set over the opening and the apparatus can be integrated, thereby improving the efficiency in printing foot shapes.

The invention will now be further explained with reference to the accompanying drawings.
Figure 1 is a perspective view showing one embodiment of the whole apparatus for printing the foot shape according to the present invention;
Figure 2 is a vertical sectional view showing a portion of a top plate;
Fig. 3 is a sectional view taken on line III-III of Fig. 2;
Fig. 4 is a diagrammatic side view of a heating means housed in a heating chamber;
Fig. 5 is a view showing the printing condition of the foot shape; and

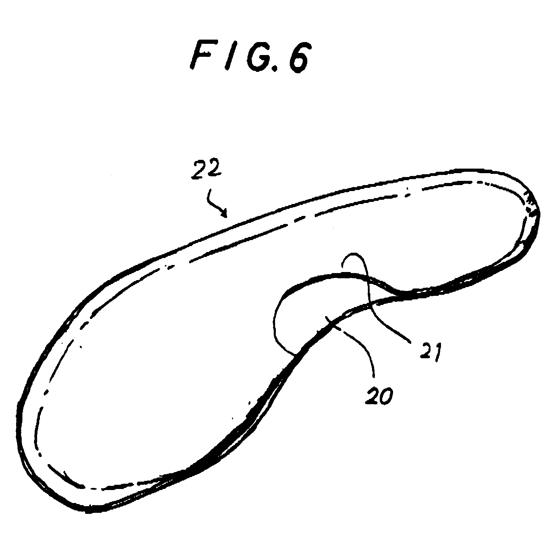

Fig. 6 is a perspective view of an insole made according to the present invention.

### Detailed Description of the Invention

In the drawings, there are shown a square printing box 1, and a cover 2 openably mounted thereto and having a top plate 3 where an opening of a predetermined area is provided, and a heating chamber 5 is adjacent to one end of the printing box 1.

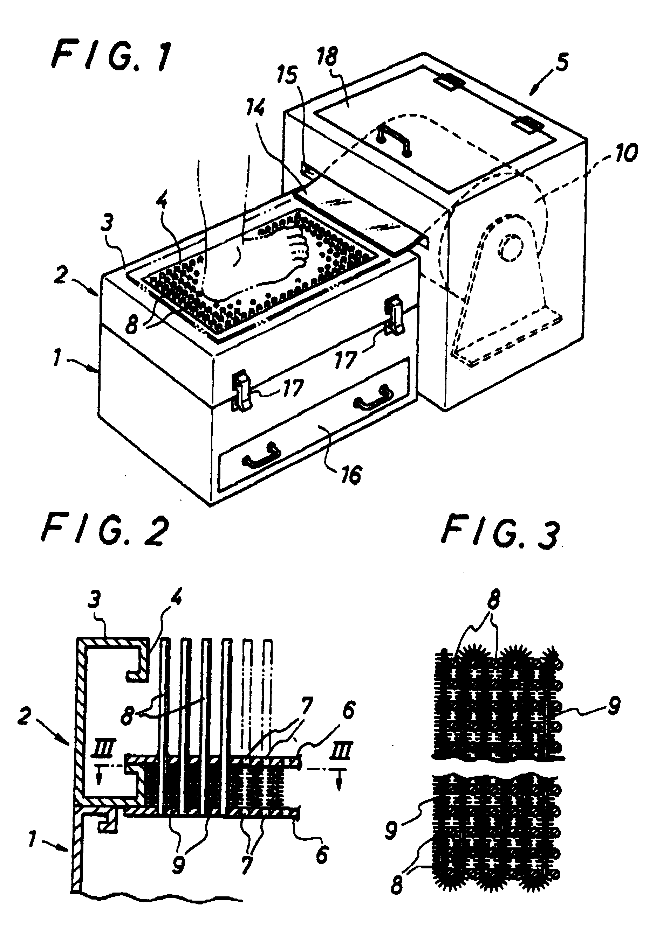

The cover 2 has therein a pair of fixed, upper and lower, holding plates 6 having a plurality of holes 7 aligned vertically. Bar-like measuring elements 8 are supported in the holes 7 so that they are individually vertically movable, the upper end of each measuring element 8 facing the opening 4. To support the measuring elements 8 individually, a tape 9 napped on both surfaces such as combined-weave velveteen is inserted in a space defined by rows of measuring elements 8 and the upper and lower holding plates 6, as shown in Figs. 2 and 3. The mechanism for supporting the measuring elements is not limited to the embodiment shown.

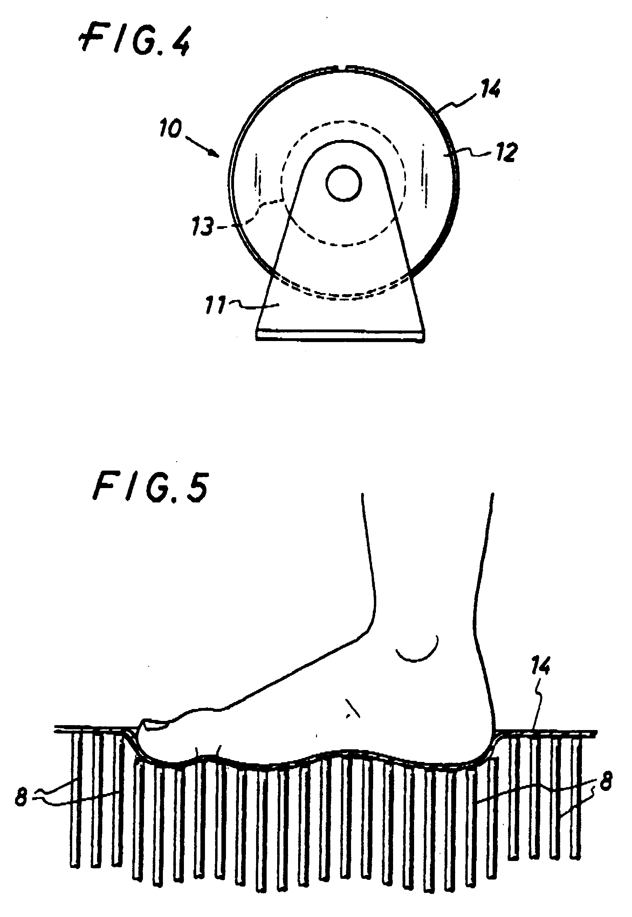

A heating means 10 is housed in the heating chamber 5 and, as shown in Fig. 4, comprises a drum 12 mounted rotatable by a stay 11 within the heating chamber 5, and a heater 13 mounted in the center of the drum 12. When the heater 13 is electrically heated by an order from a control box, not shown, the radiant heat from the heater 13 heats the drum 12 and a film 14 attached therearound which is made of a thermoplastic resin. Reference numeral 15 indicates an aperture for pulling out the film 14. Reference numeral 16 indicates a drawer for storing the film 14 provided at the bottom of the printing box 1. Reference numerals 17 indicate locks for the cover 2, and reference numeral 18 indicating a cover for the heating chamber 5.

In order to print a foot shape with the above-constructed apparatus, the cover 18 of the heating chamber 5 is opened to attach the film 14 on the surface of the drum 12 and a heat switch in the control box is turned on. Thereupon, the control box emits the order for electrically heating the heater 13, from which the radiant heat heats the drum equally and raises the ambient temperature in the heating chamber 5. Consequently, when a certain period of time passes after turning on the heat switch, the film 14 on the drum 12 is heated equally and plasticized.

After the film 14 is heated enough, it is pulled out from the pull-out aperture 15 and covered on the upper end surfaces of the measuring elements 8 facing the opening 4, whereupon a foot steps on the upper surface of the film to press it down.

Among the measuring elements 8, only those under the film contacting with the sole of the foot are lowered while the remaining measuring elements keep their original positions, and that the lowered measuring elements are pressed down in accordance with the indentation in the sole of the foot. When stepping on the film 14 which is being plasticized, it is pressed between the sole of the foot and the upper end surfaces of the measuring elements 8, therefore the foot shape is printed on the film 14.

If it is necessary to heat the film to a degree hot to the foot, the film 14 may be covered with a suitable cloth and so on when stepping thereon.

When the film is exposed to the air and lowers in temperature, it loses its plasticity and is hardened. With use of a last based on the printed foot shape or with use of this foot shape, a shoe that fits the foot can be made.

By choosing such material for the film that shows a proper elasticity after losing plasticity, the film printed with the foot shape can be cut along the outline of the foot to make an insole material 20. Thus obtained insole material 20 may be lined on its upper surface or both surfaces with a flexible or elastic covering 21 to make an insole 22 which provides a sufficient comfortableness.

Upon completion of the above printing operation, the cover 2 of the printing box 1 is opened and the measuring elements are lifted up to their original elevation to make them ready for another operation.

In lifting these measuring elements 8, they may be pushed up at their lower ends with a plate, not shown, until they are aligned with the bottom surface of the lower holding plates 6. In case of inserting the two-face napped tape 9 between adjacent rows of the measuring elements 8, as shown in the embodiment, each measuring element 8 can have necessary and sufficient resistance to ascending and descending movements while retaining its independency, so that it will not be inadvertently accompanied with the adjacent measuring element 8.

## Claims

1. An apparatus for printing a foot shape, comprising a printing box (1) with a top plate (3) having therein an opening (4) of a predetermined area, a plurality of bar-like measuring elements (8) adjacent to each other and supported in an individually vertically movable condition within said opening (4), a thermoplastic film (14) to be disposed on the upper end surfaces of said measuring elements (8) embedded in said opening so as to cover the same, and heating means (5) for plasticizing said film (14) by heating the same, said measuring elements (8) being guided in aligned holes (7) provided in two holding plates (6) extending parallel to each other in said printing box (1) and defining a space therebetween in which velveteen tapes (9) for frictionally individually engaging said measuring elements (8) are secured.

2. An apparatus as set forth in claim 1, wherein said heating means comprises a heating chamber (5) facing said opening (4), for heating said film (14) in said heating chamber (5) to impart plasticity thereto before disposing said film (14) on the upper end surfaces of said measuring elements.

3. An apparatus as set forth in claim 2, wherein said heating chamber (5) comprises a heating drum (10) for temporarily supporting said film (14).

## Patentansprüche

1. Gerät zum Abdruck einer Fußform, enthaltend einen Abdruck-Kasten (1) mit einer Deckplatte (3), die eine Öffnung (4) von einer vorbestimmten Fläche aufweist, mehrere stiftartige Meßelemente (8), die einander benachbart sind und innerhalb der Öffnung (4) in einem einzeln vertikal beweglichen Zustand gehalten sind, eine thermoplastische Folie (14), die dafür bestimmt ist, auf die oberen Stirnflächen der in die Öffnung eingebetteten Meßelemente (8) gelegt zu werden, um diese zu bedecken, und eine Heizeirrichtung (5) zum Plastifizieren der Folie (14) durch Erwärmen derselben, wobei die Meßelemente (8) in ausgerichteten Löchern (7) geführt sind, die in zwei Halteplatten (6) vorgesehen sind, die sich im Abdruck-Kasten (1) parallel zueinander erstrecken und einen Zwischenraum abgrenzen, in dem Baumwollsamt-Bänder (9) befestigt sind, die durch Reibung die Meßelemente (8) einzeln halten.

2. Gerät nach Anspruch 1, wobei die Heizeinrichtung eine Heizkammer gegenüber der Öffnung (4) zum Aufheizen genannter Folie (14) in genannter Herzkammer (5) miteinschließt, um ihr Verformbarkeit zu verleihen, bevor die Folie (14) auf die oberen Stirnflächen der Meßelemente gelegt wird.

3. Gerät nach Anspruch 2, wobei die Heizkammer (5) eine Heiztrommel (10) zum zeitweiligen Halten der Folie (14) enthält.

## Revendications

1. Un appareil pour faire l'empreinte d'une forme de pied, comportant une boîte d'empreinte (1) avec une plaque supérieure (3) présentant intérieurement une ouverture (4) d'une aire prédéterminée, une pluralité d'éléments de mesure (8) en forme de barre adjacents l'un à l'autre et supportés de manière à être déplaçables individuellement verticalement dans ladite ouverture (4), une pellicule thermoplastique (14) destinée à être disposée sur les surfaccs extrêmes supérieures desdits éléments de mesure (8) logés dans ladite ouverture pour recouvrir ceux-ci, et des moyens de chauffage (5) pour plastifier ladite pellicule (14) en la chauffant, lesdits éléments de mesure (8) étant guidés dans des alésages alignés (7) prévus dans deux plaques de support (6) s'étendant parallèlement l'une à l'autre dans ladite boîte d'empreinte (1) et définissant entre elles un espace dans lequel sont assujetties des bandes (9) du type velours pour coopérer individuellement par friction avec lesdits éléments de mesure (8).

2. Un appareil selon la revendication 1, dans lequel lesdits moyens de chauffage comportent une chambre de chauffage (5) en regard de ladite ouverture (4), pour chauffer ladite pellicule (14) dans ladite chambre de chauffage (5) de manière à donner à celle-ci une plasticité avant le dépôt de ladite pellicule (14) sur les surfaces extrêmes supérieures desdits éléments de mesure.

3. Un appareil selon la revendication 2, dans lequel ladite chambre de chauffage (5) comporte un tambour de chauffage (10) pour supporter temporairement ladite pellicule (14).
